(19) **Europäisches Patentamt**
**European Patent Office**
**Office européen des brevets**

(11) **EP 1 687 249 B1**

(12) **EUROPÄISCHE PATENTSCHRIFT**

(45) Veröffentlichungstag und Bekanntmachung des Hinweises auf die Patenterteilung:
**02.12.2009 Patentblatt 2009/49**

(21) Anmeldenummer: **04763312.8**

(22) Anmeldetag: **17.07.2004**

(51) Int Cl.:
*C07C 45/35* *(2006.01)*      *C07C 45/90* *(2006.01)*
*C07C 51/64* *(2006.01)*      *C07C 47/22* *(2006.01)*
*C07C 57/04* *(2006.01)*

(86) Internationale Anmeldenummer:
**PCT/EP2004/008012**

(87) Internationale Veröffentlichungsnummer:
**WO 2005/016852 (24.02.2005 Gazette 2005/08)**

(54) **VERFAHREN ZUM BETREIBEN EINER KONTINUIERLICHEN HETEROGEN KATALYSIERTEN GASPHASEN-PARTIALOXIDATION WENIGSTENS EINER ORGANISCHEN VERBINDUNG**

METHOD FOR OPERATING A CONTINUOUS HETEROGENEOUSLY CATALYSED GAS-PHASE PARTIAL OXIDATION OF AT LEAST ONE ORGANIC COMPOUND

PROCEDE POUR REALISER UNE OXYDATION PARTIELLE CONTINUE EN PHASE GAZEUSE, CATALYSEE DE MANIERE HETEROGENE, D'AU MOINS UN COMPOSE ORGANIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **06.08.2003 DE 10336385**
**06.08.2003 US 492726 P**
**19.12.2003 DE 10360396**
**19.12.2003 US 530616 P**

(43) Veröffentlichungstag der Anmeldung:
**09.08.2006 Patentblatt 2006/32**

(73) Patentinhaber: **BASF SE**
**67056 Ludwigshafen (DE)**

(72) Erfinder:
• **SCHLIEPHAKE, Volker**
**67105 Schifferstadt (DE)**
• **HAMMON, Ulrich**
**68163 Mannheim (DE)**
• **BECHER, Rolf-Dieter**
**68199 Mannheim (DE)**
• **MÜLLER-ENGEL, Klaus, Joachim**
**79297 Stutensee (DE)**

(56) Entgegenhaltungen:
**EP-A1- 0 990 636**

**Beschreibung**

Die vorliegende Erfindung betrifft ein Verfahren zum Betreiben einer kontinuierlichen heterogen katalysierten Gasphasen-Partialoxidation wenigstens einer organischen Verbindung in einem Oxidationsreaktor, dessen Beschickungsgasgemisch neben der wenigstens einen partiell zu oxidierenden Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfasst, bei dem sowohl als Sauerstoff - als auch als Inertgasquelle für das Beschickungsgasgemisch Luft mitverwendet wird, die zuvor in einem Verdichter von einem niederen Anfangsdruck auf einen höheren Enddruck verdichtet worden ist.

Unter einer vollständigen Oxidation einer organischen Verbindung mit molekularem Sauerstoff wird hier verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidation einer organischen Verbindung zusammengefasst. D.h., der Begriff der Partialoxidation soll in dieser Schrift insbesondere auch partielle Ammoxidationen umfassen, die dadurch charakterisiert sind, dass die partielle reaktive Umsetzung der organischen Verbindung im Beisein von Ammoniak erfolgt.

Im besonderen sollen hier unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält, als vor Durchführung der Partialoxidation.

Als ein sich unter den Bedingungen der heterogen katalysierten Gasphasen-Partialoxidation im wesentlichen inert verhaltendes Verdünnungsgas werden solche Verdünnungsgase verstanden, deren Bestandteile unter den Bedingungen der heterogen katalysierten Gasphasen-Partialoxidation - jeder Bestandteil für sich betrachtet - zu mehr als 95 mol%, vorzugsweise zu mehr als 99 mol% unverändert erhalten bleiben.

Es ist allgemein bekannt, dass durch heterogen katalysierte partielle Oxidation verschiedener organischer Vorläuferverbindungen mit molekularem Sauerstoff in der Gasphase zahlreiche Grundchemikalien erzeugt werden können.

Beispielhaft genannt seien die Umsetzung von Xylol zu Phthalsäureanhydrid, die Umsetzung von Propylen zu Acrolein und/oder Acrylsäure (vgl. z.B. DE-A 2351151), die Umsetzung von tert.-Butanol, iso-Buten, iso-Butan, iso-Butyraldehyd oder dem Methylether des tert.-Butanols zu Methacrylnitril oder zu Methacrolein und/oder Methacrylsäure (vgl. z.B. DE-A 2526238, EP-A 92097, EP-A 58927, DE-A 4132263, DE-A 4132684 und DE-A 4022212), die Umsetzung von Acrolein zu Acrylsäure, die Umsetzung von Methacrolein zu Methacrylsäure (vgl. z.B. DE-A 2526238), die Umsetzung von Butadien zu Maleinsäureanhydrid (vgl. z.B. die DE-A 2106796 und die DE-A 1624921), die Umsetzung von n-Butan zu Maleinsäureanhydrid (vgl. z.B. GB-A 1464198 und GB-A 1291354), die Umsetzung von Ethylen zu Ethylenoxid oder von Propylen zu Propylenoxid (vgl. z.B. DE-AS 1254137, DE-A 2159346, EP-A 372972, WO 89/0710, DE-A 4311608 und Beyer, Lehrbuch der organischen Chemie, 17. Auflage (1973), Hirzel Verlag Stuttgart, S. 261), die Umsetzung von Propylen und/oder Acrolein zu Acrylnitril (vgl. z.B. DE-A 2351151), die Umsetzung von iso-Buten und/oder Methacrolein zu Methacrylnitril, die oxidative Dehydrierung von Kohlenwasserstoffen (vgl. z.B. DE-A 2351151), die Umsetzung von Propan zu Acrylnitril oder zu Acrolein und/oder Acrylsäure (vgl. z.B. DE-A 10131297, EP-A 1090684, EP-A 608838, DE-A 10046672, EP-A 529853, WO 01/96270 und DE-A 10028582) etc.. Insbesondere auf alle vorgenannten Partialoxidationen ist das erfindungsgemäße Verfahren anwendbar.

Bei den für solche Reaktionen zu verwendenden Katalysatoren handelt es sich normalerweise um Festkörper.

Besonders häufig handelt es sich bei den zu verwendenden Katalysatoren um feste Oxidmassen oder Edelmetalle (z.B. Ag). Die katalytisch aktive Oxidmasse kann neben Sauerstoff lediglich ein anderes Element oder mehr als ein anderes Element (Multielementoxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen.

Aufgrund des in der Regel ausgeprägt exothermen Charakters der meisten heterogen katalysierten Gasphasen-Partialoxidationen organischer Verbindungen mit molekularem Sauerstoff werden die Reaktionspartner üblicherweise mit einem sich unter den Bedingungen der heterogen katalysierten Gasphasen-Partialoxidation im wesentlichen inerten Gas verdünnt, das mit seiner Wärmekapazität frei werdende Reaktionswärme zu absorbieren vermag und die Reaktionsgeschwindigkeit günstig beeinflusst.

Eines der häufigsten mitverwendeten inerten Verdünnungsgase ist molekularer Stickstoff, der automatisch immer dann zur Anwendung kommt, wenn als Sauerstoffquelle für die heterogen katalysierte Gasphasen-Partialoxidation Luft mitverwendet oder ausschließlich verwendet wird.

Ein anderes vielfach mitverwendetes inertes Verdünnungsgas ist wegen seiner allgemeinen Verfügbarkeit Wasserdampf. Vielfach wird auch Kreisgas (das in der Regel noch nicht verbrauchten Sauerstoff enthält) als inertes Verdünnungsgas mitverwendet (vgl. z.B. EP-A 1180508). Als Kreisgas wird das Restgas bezeichnet, das nach einer einstufigen oder mehrstufigen (bei der mehrstufigen heterogen katalysierten Gasphasen-Partialoxidation organischer Verbindungen wird die Gasphasen-Partialoxidation im Unterschied zur einstufigen heterogen katalysierten Gasphasen-Partialoxidation nicht in einem, sondern in wenigstens zwei hintereinandergeschalteten Reaktoren durchgeführt, wobei zwischen aufeinanderfolgenden Reaktoren Oxidationsmittel (z.B. in Form von Luft) ergänzt werden kann; die Mehrstufigkeit wird insbesondere dann angewendet, wenn sich die Partialoxidation in aufeinanderfolgenden Schritten vollzieht; in diesen Fällen ist es häufig zweckmäßig, sowohl den Katalysator als auch die sonstigen Reaktionsbedingungen an den jeweiligen Reaktionsschritt optimierend anzupassen, und den Reaktionsschritt in einem eigenen Reaktor, in einer separaten Reaktionsstufe durchzuführen; sie kann aber auch dann angewendet werden, wenn aus Gründen der Wärmeabfuhr oder aus anderen Gründen (vgl. z.B. DE-A 19902562) der Umsatz auf mehrere hintereinandergeschaltete Reaktoren verschmiert wird; ein Beispiel für eine häufig zweistufig durchgeführte heterogen katalysierte Gasphasen-Partialoxidation ist die Partialoxidation von Propylen zu Acrylsäure; in der ersten Reaktionsstufe wird das Propylen zum Acrolein, und in der zweiten Reaktionsstufe das Acrolein zu Acrylsäure oxidiert; in entsprechender Weise wird häufig auch die Methacrylsäureherstellung (über Methacrolein als Zwischenprodukt), meist ausgehend von iso-Buten, zweistufig durchgeführt; beide vorgenannten Partialoxidationen können bei Verwendung geeigneter Katalysatorbeschickungen aber auch einstufig (beide Schritte in einem Reaktor) durchgeführt werden; vgl. z.B. EP-A 990636 und EP-A 1106598) heterogen katalysierten Gasphasen-Partialoxidation wenigstens einer organischen Verbindung dann verbleibt, wenn man aus dem Produktgasgemisch das Zielprodukt mehr oder weniger selektiv (z.B. durch Absorption in ein geeignetes Lösungsmittel; vgl. z.B. DE-A 19606877) abtrennt. Im Regelfall besteht es überwiegend aus den für die Partialoxidation verwendeten inerten Verdünnungsgasen sowie aus bei der Partialoxidation üblicherweise als Nebenprodukt gebildetem Wasserdampf und durch unerwünschte vollständige Neben-Oxidation gebildeten Kohlenoxiden. Teilweise enthält es noch geringe Mengen an bei der Partialoxidation nicht verbrauchtem Sauerstoff (Restsauerstoff) und/oder an nicht umgesetzten organischen Ausgangsverbindungen. Üblicherweise wird nur eine Teilmenge des Restgases als Kreisgas verwendet. Die verbleibende Restgasmenge wird normalerweise verbrannt.

Die Durchführung einer heterogen katalysierten Gasphasen-Partiatoxidation erfolgt normalerweise an einem Katalysatorfest- oder in einem Katalysatorwirbelbett.

Dazu wird das Reaktionsgasausgangsgemisch, das im wesentlichen aus der wenigstens einen partiell zu oxidierenden organischen Verbindung (üblicherweise Vorläuferverbindung genannt), molekularem Sauerstoff (gegebenenfalls Ammoniak im Fall einer Ammoxidation) und inertem Verdünnungsgas (einschließlich gegebenenfalls Kreisgas) besteht, in der Regel bei erhöhter Temperatur (in der Regel einige hundert °C, üblicherweise 100 bis 600°C) durch die Katalysatorbeschickung geführt. Die chemische Umsetzung erfolgt während der Kontaktzeit an der Katalysatoroberfläche.

Wie bei der Kreisgasbildung bereits erwähnt, wird aufgrund zahlreicher im Verlauf der katalytischen Gasphasenpartialoxidation erfolgender Parallel- und Folgereaktionen sowie aufgrund der in der Regel mitzuverwendenden inerten Verdünnungsgase (unter besonderen Umständen kann auch die wenigstens eine organische Vorläuferverbindung als Verdünnungsgas fungieren; nämlich dann, wenn sie im Reaktionsgasausgangsgemisch relativ zum in selbigem enthaltenem molekularen Sauerstoff im Überschuss vorliegt) bei einer heterogen katalysierten Gasphasenpartialoxidation keine reine organische Zielverbindung sondern ein Reaktionsgasgemisch erhalten, aus welchem das Zielprodukt abgetrennt werden muss.

Bildet der Bereich der Gasphasenoxidation die eigentliche Reaktionszone, wird das Produktgasgemisch zum Zweck der Zielproduktabtrennung normalerweise einer sogenannten Aufarbeitungszone zugeführt, in welcher diese Abtrennung erfolgt.

In typischer Weise (z.B. im Fall von Acrylsäure und im Fall von Methacrylsäure) erfolgt die Zielproduktabtrennung aus dem Produktgasgemisch über extraktive, fraktionierend kondensierende und/oder rektifikative Trennverfahren in trennwirksame Einbauten enthaltenden Trennkolonnen, durch die das Produktgasgemisch geführt wird (vgl. z.B. DE-A 19606877, DE-A 19631645, EP-A 982289, DE-A 19740253, EP-A 982287, EP-A 1 041 062, EP-A 778 255, EP-A 695 736, DE-A 19 501 325 und EP-A 925 272). Das dabei verbleibende Restgas wird, wie bereits beschrieben, bei Bedarf als Kreisgas zur Verdünnung des Reaktionsgasausgangsgemischs mitverwendet.

Zur Förderung des Reaktionsgasgemisches durch die Katalysatorbeschickung der heterogen katalysierten partiellen Gasphasenoxidation sowie durch die sich daran anschließende Aufarbeitung bedarf es eines Druckunterschiedes zwischen Reaktoreingang und Restgasausgang.

Dieser Druckunterschied wird in der Praxis üblicherweise dadurch erzeugt, dass das Reaktionsgasausgangsgemisch vorab seines Eintritts in den Oxidationsreaktor auf einen gegenüber dem Luftdruck der Umgebung erhöhten Druck eingestellt wird. Diese Drucke betragen in typischer Weise 0,2 bis 5 barü (ü steht für Überdruck gegenüber gewöhnlichem Atmosphärendruck) oder mehr, häufig 0,5 bis 4,5 barü, und vielfach 1 oder 2 bis 4 barü. Hohe Drucke werden dabei insbesondere dann benötigt, wenn der zu fördernde Gasvolumenstrom groß ist (z.B. bei Hochlastfahrweisen, wie sie in den Schriften DE-A 19927624, DE-A 19948248, DE-A 19948241, DE-A 19910508, DE-A 10313210, DE-A 10313214, DE-A 10313213 und DE A 19910506 beschrieben sind), da letzteres bei gegebenem Reaktor und gegebener Aufarbeitungsvorrichtung auch einen erhöhten Druckverlust bei der Förderung durch die Katalysatorbeschickung, gegebenenfalls mit Füllkörpern beschickte Zwischen- und/oder Nachkühler sowie die Aufarbeitungsapparate bedingt.

Während die partiell zu oxidierende organische Vorläuferverbindung in der Praxis häufig flüssig gelagert wird, genügt in der Regel einfaches Verdampfen, um die partiell zu oxidierende organische Vorläuferverbindung auf den erforderlichen Reaktoreingangsdruck zu bringen. Als inertes Verdünnungsgas mitzuverwendender Wasserdampf steht aus den unterschiedlichsten Quellen meistens ebenfalls mit ausreichendem überatmosphärischem Druck zu Verfügung.

Dies trifft in aller Regel jedoch nicht für als Sauerstoffquelle mitverwendete Luft (sie wird üblicherweise mit Atmosphärendruck der den Oxidationsreaktor umgebenden Atmosphäre entnommen), das Kreisgas (es weist normalerweise den Reaktoreingangsdruck abzüglich des Druckverlustes auf dem Weg durch die Oxidations- und durch die Aufarbeitungszone auf) und gegebenenfalls sonstige inerte Verdünnungsgase zu.

In der Praxis ist es daher normalerweise erforderlich, wenigstens die als Sauerstoffquelle mitverwendete Luft mittels eines Verdichters von einem niederen Anfangsdruck auf einen höheren Enddruck (meist den Reaktoreingangsdruck) zu bringen (vgl. z.B. Fig. 1 der EP-A 990636).

Dabei kann die Verdichtung dieser Bestandteile (z.B. die Sauerstoffquelle Luft und die Verdünnungsgasquelle Kreisgas) in räumlich getrennten Verdichtern oder in einem einzigen Verdichter (vgl. Fig. 1 der EP-A 990636) erfolgen. Bei Bedarf können über einen Luftverdichter auch mehrere Verfahren der heterogen katalysierten Gasphasen-Partialoxidation mit verdichteter Luft (z.B. über entsprechende Zuführleitungen) versorgt werden.

Die aus verschiedenen Quellen stammenden, im wesentlichen auf Reaktoreingangsdruck befindlichen (gebrachten) Teilmengen des Beschickungsgasgemisches (Reaktionsgasausgangsgemisches) werden dann aus getrennten Leitungen kommend zunächst in einem z.B. statischen Mischer (in der Regel Raum mit Einbauten, die Turbulenzen erzeugen) vermischt, und nachfolgend gegebenenfalls auf Eingangstemperatur erwärmt und dann dem Oxidationsreaktor zugeführt (der Eintritt der Einzelgase in die dem statischen Mischer zugeführte Leitung wird dabei in zweckmäßiger Weise so gewählt (sowohl in Abfolge als auch Menge), dass das Entstehen explosiver Mischungen vermieden wird (im Fall einer Partialoxidation von Propylen zu z.B. Acrolein und/oder Acrylsäure könnte diese Eintrittsabfolge in zweckmäßiger Weise z.B. zunächst Kreisgas und/oder Dampf, dann Roh-Propen und dann Luft lauten.

Prinzipiell können zum Verdichten von Gasen Verdichter unterschiedlichster Art eingesetzt werden. Beispielhaft aufgeführt seien Verdrängungsverdichter (z.B. Hubkolbenverdichter, Schraubenverdichter und Drehkolbenverdichter), Strömungsverdichter (z.B. Turboverdichter, Kreiselverdichter, Axialverdichter und Radialverdichter) und Strahlverdichter.

Besonders geeignete Radialverdichter sind z.B. die Verdichter GV10/3L der Fa. Gutehoffnungshütte (GHH), die Verdichter GS900 und GKS450 der Fa. Borsig, der Verdichter VK80-2 der Fa. Mannesmann Demag oder der Verdichter SRL1001/B der Fa. Nuovo Pignone.

Die Funktionsweise eines Radialverdichters lässt sich wie folgt verdeutlichen (vgl. auch DE-A 10259023):

Er besteht im Prinzip aus einem Gehäuse und wenigstens einem in selbigem rotierenden, von einer Antriebswelle angetriebenen, Laufrad, das mit Schaufeln versehen ist. Das zu verdichtende Gas tritt axial durch einen Saugstutzen ein. Es wird durch die Fliehkraft mittels des rotierenden Laufrades (geschlossene Scheibe mit Schaufeln) radial nach außen gelenkt und vom Laufrad auf diesem Weg auf hohe Geschwindigkeit beschleunigt. Das Gehäuse hat die Aufgabe, das Gas aufzufangen, damit es gesammelt durch die Druckausgänge weitergeleitet werden kann. Das Gehäuse hat gleichzeitig die Aufgabe, Bewegungsenergie in Druck umzuwandeln. Dazu wird in der Regel ausgenutzt, dass eine Querschnittsvergrößerung die Geschwindigkeit des Gases herabsetzt und dadurch einen Anstieg des statischen Druckes bewirkt. Zur Querschnittsvergrößerung sind unterschiedliche konstruktive Ausführungen des Gehäuses möglich. Bei einstufigen Verdichtern oder hinter der letzten Stufe mehrstufiger Verdichter kommen häufig Spiralgehäuse zur Anwendung. Dieses umschließt das Laufrad in Spiralform. Der Querschnitt erweitert sich in Richtung auf den Druckausgang. Das durchfließende Gas wird dadurch verlangsamt, was eine gleichzeitige Druckzunahme bedeutet.

Anstelle der Spirale können, besonders bei mehrstufigen (z.B. 1- bis 3-stufigen) Verdichtern, auch feststehende Leiträder verwendet werden. Das Leitrad ist im Gehäuse eingebaut und als Ringraum ausgebildet. Es umschließt das Laufrad. Im Leitrad sind Leitschaufeln angeordnet, die zueinander sich nach außen hin stetig erweiternde Kanäle bilden. Bei dieser Ausführung wird das Gas nicht direkt in das Gehäuse geschleudert, sondern es durchfließt zunächst die Schaufelkanäle des Leitrads. Durch die Erweiterung in Fließrichtung bewirken sie wiederum eine Verlangsamung der Fließgeschwindigkeit und den dadurch bedingten Druckaufbau.

Bei mehrstufigen Verdichtern kann in vorteilhafter Weise hinter jeder Verdichtungsstufe verdichtetes Gas (z.B. Luft) entnommen werden. Dies ermöglicht dann z.B. eine besonders wirtschaftliche Verdichtung, wenn das zu verdichtende Gas auf unterschiedlichen Druckstufen benötigt wird. Letzteres ist z.B. bei mehrstufigen Partialoxidationen am Katalysatorfestbett mit Zwischenlufteinspeisung (hinter der ausgeführten Oxidationsstufe) der Fall (z.B. bei der zweistufigen Partialoxidation von Propylen zu Acrylsäure). Es kann aber auch dann zweckmäßig sein, wenn eine Teilmenge der verdichteten Luft parallel für Stripanwendungen (benötigt geringeres Druckniveau) entnommen wird.

Die EP-A 990636 läßt die Frage der Art des zu verwendenden Verdichters völlig offen. Sie lehrt jedoch, dass die als Sauerstoffquelle mitzuverwendende Luft vorab ihrer Verdichtung lediglich einer thermischen Behandlung bedarf.

In der DE-A 10259023 wird selbst eine vorab der Verdichtung durchzuführende thermische Behandlung von als Sauerstoffquelle mitzuverwendender Luft für nicht erforderlich erachtet. Vielmehr wird empfohlen, als Verdichter einen Radialverdichter zu verwenden, da dieser gegenüber feinstpartikelförmigen festen oder flüssigen Bestandteilen im zu verdichtenden Gas weitgehend unempfindlich sei. Dies vor allem vor dem Hintergrund, dass bei einer Beteiligung von wenigstens eine ethylenisch ungesättigte Doppelbindung aufweisenden chemischen Verbindungen ("Monomeren") an der heterogen katalysierten Gasphasen-Partialoxidation das Kreisgas in der Regel auch Monomeren haltig ist (als eine ethylenisch ungesättigte Doppelbindung soll hier eine chemische Doppelbindung zwischen zwei Kohlenstoffatomen verstanden werden, die im Molekül entweder singulär, von anderen Mehrfachbindungen isoliert oder zu anderen Mehrfachbindungen konjugiert oder anneliert sein kann; eine solche Doppelbindung aufweisende chemische Verbindung ist in die meisten heterogen katalysierten Gasphasen-Partialoxidationen (z.B. in nahezu alle Eingangs zitierten) involviert; sie kann z.B. die partiell zu oxidierende organische Vorläuferverbindung (z.B. Butadien, Propylen, iso-Buten, Acrolein, Methacrolein), oder das Zielprodukt (z.B. Acrylsäure, Methacrylsäure, Acrylnitril, Methacrylnitril) oder ein Zwischenprodukt (z.B. Acrolein oder Methacrolein) sein). Insbesondere im Fall einer gemeinsamen Verdichtung von Luft und Kreisgas (gemäß der DE-A 10259023 kann die Verdichtung des Kreisgases und der Luft in zwei getrennten Radialverdichtern, die mit zwei getrennten Motoren angetrieben werden, oder in zwei Verdichtern die mit einem Motor angetrieben werden oder in einem einzigen mit einem Motor angetriebenen Verdichter durchgeführt werden) ist eine von solchen Restmonomeren ausgehende Polymerpartikelbildung beim Verdichten weitgehend unvermeidbar.

Detaillierte Analysen von Langzeitversuchen haben jedoch ergeben, dass sowohl die Empfehlung der DE-A 10259023 als auch die der EP-A 990636 nicht voll zu befriedigen vermögen. Vielmehr wurde in überraschender Weise gefunden, dass in als Sauerstoffquelle mitverwendeter Luft in winzigsten Mengen enthaltene feste und/oder flüssige Feinstpartikel (mit einer Längstauschdehnung von in der Regel $\leq 100$ $\mu$m, häufig $\geq 0,1$ bzw. $\geq 0,2$ bis 50 $\mu$m) sich langfristig doch in nicht vernachlässigbarer Weise sowohl bei der Luftverdichtung (selbst wenn diese gemeinsam mit Restmonomere haltigem Kreisgas in einem Verdichter durchgeführt wird) als auch bei der Ausbildung von über die Betriebsdauer zunehmendem Druckverlust bei der Durchführung der Gasphasen-Partialoxidation an einem Katalysatorfestbett nachteilig auswirken. Zusätzlich wirken sie sich nachteilig auf die Katalysatorperformance (Aktivität und/oder Selektivität) aus.

Die Aufgabe der vorliegenden Erfindung bestand daher darin, ein Verfahren zum Betreiben einer kontinuierlichen heterogen katalysierten Gasphasen-Partialoxidation wenigstens einer organischen Verbindung zur Verfügung zu stellen, das den Nachteilen der Verfahren des Standes der Technik weitgehend abhilft.

Demgemäß wurde ein Verfahren zum Betreiben einer kontinuierlichen heterogen katalysierten Gasphasen-Partialoxidation wenigstens einer organischen Verbindung in einem Oxidationsreaktor, dessen Beschickungsgasgemisch neben der wenigstens einen partiell zu oxidierenden Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfasst, bei dem sowohl als Sauerstoff- als auch Inertgasquelle für das Beschickungsgasgemisch Luft mit verwendet wird, die zuvor in einem Verdichter von einem niederen Anfangsdruck auf einen höheren Enddruck verdichtet worden ist, gefunden, das dadurch gekennzeichnet ist, dass die Luft vorab ihres Eintritts in den Verdichter wenigstens einer mechanischen Trennoperation unterworfen wird, mit der in der Luft dispergierte Feststoffpartikel abgetrennt werden können.

Bei erfindungsgemäß geeigneten, eine mechanische Trennoperation anwendenden, Luftreinigungsverfahren wird normalerweise durch äußere Kräfte eine Relativbewegung der dispergierten Feststoffpartikel (und/oder Flüssigpartikel) gegenüber dem Trägergas erzeugt. Je nach den hauptsächlich wirkenden Kräften werden dabei z.B. folgende Abscheideprinzipien unterschieden:

- Prall-, Stoß- und Fliehkräfte in Umlenkabscheidern,
- Zentrifugalkräfte in Fliehkraftabscheidern,
- Prallwirkung und Haftkräfte in filternden Abscheidern,
- elektrostatische Kräfte in Elektrofiltern.

D.h., erfindungsgemäß geeignete, eine mechanische Trennoperation anwendende, Gasreinigungsapparate sind z.B. Kammer-, Prall- und Zentrifugalabscheider, die Massekräfte nutzen. Es sind für das erfindungsgemäße Verfahren aber auch akustische Abscheider anwendbar. Bevorzugt sind Aerozyklone. In einfacher Weise kann erfindungsgemäß als mechanische Trennoperation aber auch filtriert werden.

Als Filterschichten kommen z.B. Filtergewebe, poröse Filtermassen, Papiervlies oder ölbenetzte Metallfilter in Betracht. Auch Elektroabscheider sind erfindungsgemäß anwendbar. In einfachster Weise kann die zu filtrierende Luft auch ein inertes Festbett durchströmen, indem sich in der Luft enthaltene feinste feste (und/oder flüssige) Partikel abscheiden, bevor die Luft den Verdichter erreicht. Der Begriff der mechanischen Trennoperation soll in dieser Schrift auch Sprühvorrichtungen umfassen, in denen die Luft im Gleich- oder im Gegenstrom Flüssigkeitströpfchen ausgesetzt wird (z.B. von hochsiedenden organischen Flüssigkeiten oder von Wasser), die in der Luft enthaltene Feststoffpartikel aufzunehmen vermögen. Die Sprühflüssigkeit wird nach einigen Rezirkulationen ausgetauscht, um eine Sättigung mit Feststoffpartikeln zu vermeiden. Am Ende der Waschung ist zweckmäßig ein Tröpfchenabscheider angebracht.

Selbstverständlich können erfindungsgemäß auch verschiedene hintereinandergeschaltete mechanische Trennoperationen angewendet werden.

Eine erfindungsgemäß bevorzugte mechanische Trennoperation ist das Filtrieren, lassen sich damit doch auf vergleichsweise einfache Weise Teilchen mit einer Längstausdehnung von 0,001 $\mu$m und weniger noch zurückhalten. Bei entsprechender Dimensionierung und Auswahl des Filterstoffs lassen sich auf wesentlich kostengünstigere Art und Weise als beim Elektrofilter Abscheidegrade von mehr als 99,9 % erreichen.

Die Abscheidewirkung basiert beim Filtrieren im wesentlichen auf Prallwirkung (Stoß der Feinstpartikel auf das Filterelement) und Diffusion, wobei aber auch andere Faktoren, wie Gravitation und elektrostatische Kräfte von Einfluß sind. Obwohl es sich bei der Filtration um keinen reinen Siebvorgang handelt (die beim erfindungsgemäßen Verfahren durch Filtration abgeschiedenen Teilchen sind in überraschender Weise häufig wesentlich kleiner als die Poren des Filtermediums), haben engmaschige Filter beim erfindungsgemäßen Verfahren einen höheren Wirkungsgrad als weitmaschige, allerdings auf Kosten des Widerstands, d.h., der Wirtschaftlichkeit.

Anwendungstechnisch zweckmäßig können für das erfindungsgemäße Verfahren unter anderem Gewebefilter eingesetzt werden. Prinzipiell eignen sich für das erfindungsgemäße Verfahren Filtergewebe aus Natur- oder Chemiefasern. D.h., sowohl Filtergewebe aus PVC, Polyamiden (Perlon®, Nylon®), Wolle, Polyacrylnitril (Redon®, Dralon®), Polyester und Polytetrafluorethylen (Teflon®) als auch aus siliconisiertem Glasgewebe sind erfindungsgemäß geeignet. Anstelle von Geweben können erfindungsgemäß auch Wirrfaservliese derselben Materialien verwendet werden. Sie bestehen meist aus synthetischen Fasern, die z.B. mit Hilfe eines Nadelverfahrens auf ein Stützgewebe aufgetragen (z.B. Polyesterfasern auf einer Polyestergaze) oder mit Bindemitteln verfestigt werden. Erfindungsgemäß verwendbares Filtergewebe ist aber auch Baumwolle oder Leinen. Als Material für Luftfilter können ferner Drahtgestricke, Matten aus Metallspänen, Glas- oder chemischen Fasern, Asbest oder Papier verwendet werden. Zur besseren Filterung ist das Filtergewebe zur Reinluftseite hin in der Regel verdichtet. Generell eignen sich für das erfindungsgemäße Verfahren Filter, wie sie auch in großen Klima- und Lüftungsanlagen zum Einsatz kommen. Ein günstiges Brandverhalten des Filtermaterials im Sinne der DIN 53438 ist bevorzugt.

Die wichtigste Forderung für ein für das erfindungsgemäße Verfahren geeignetes Filtergewebe (Filtertuch) bzw. Faservlies ist eine möglichst große Luftdurchlässigkeit bei hohem Rückhaltevermögen.

Die Flächenbelastung mit zu filtrierendem Gas kann beim erfindungsgemäßen Verfahren typisch 5 bis 20000, häufig 500 oder 1000 bis 15000 Nm$^3$/m$^2$ · h betragen. Flächenbelastungen von 2000 bis 10000 Nm$^3$/m$^2$ h sind erfindungsgemäß bevorzugt.

Der Druckverlust (Differenz zwischen dem Druck des zu filtrierenden Gases vor dem Filter und dem Druck des zu filtrierenden Gases nach Durchgang durch das Filter) sollte bei einer Flächenbelastung von 5000 Nm$^3$/m$^2$ h erfindungsgemäß zweckmäßig für frisches Gewebe etwa 0,01 bis 10 mbar, vorzugsweise 0,05 oder 0,1 bis 5 mbar, besonders bevorzugt 0,2 bis 1 mbar betragen. Gleichzeitig sollte der Abscheidegrad bei wenigstens 75 % bzw. 85 % oder 95 %, bevorzugt bei wenigstens 97 % und besonders bevorzugt bei wenigstens 99 % liegen.

Üblicherweise wird man das Filtergewebe bzw. -vlies spätestens dann durch neues Gewebe bzw. Vlies ersetzen (oder es seiner Reinigung zuführen), wenn der Druckverlust bei vorgenannter Belastung um 10 mbar, vorzugsweise nur um 5 mbar, besonders bevorzugt nur um 2 mbar angestiegen ist.

Die Geschwindigkeit mit der die Luft, z.B. angesaugt durch einen Radialverdichter, auf das Filtergewebe bzw. -vlies zuströmt, beträgt beim erfindungsgemäßen Verfahren häufig 0,5 bis 3 m/s.

Erfindungsgemäß wesentlich ist, dass das Filtergewebe bzw. -vlies sich unter der Belastung mit zu filtrierendem Gas nicht wesentlich ausdehnt.

Darüber hinaus sollte sich die Dehnbarkeit des Filtergewebes bzw. -vlies im Temperaturbereich von etwa -30°C bis +50°C (typische mögliche Außentemperaturen) nicht wesentlich ändern. Insbesondere sollte das Filtergewebe bzw. -vlies bei den möglichen tiefen Außentemperaturen nicht spröde werden.

Als erfindungsgemäß besonders günstig haben sich Filtervliese aus auf Polyestergaze genadelten Polyesterfasern erwiesen, die bei einer Flächenbelastung von 5000 Nm$^3$/m$^2$ h und einem Druckverlust von 0,1 bis 5 mbar im frischen Zustand die Abscheidegrade gemäß DIN EN 779 (Filterklasse G3) erzielen. Diese können z.B. sein:

| Partikelgröße | Abscheidegrad (% bezogen auf die Gesamtpartikelanzahl der jeweiligen Partikelgröße) |
|---|---|
| $\leq 0,5\ \mu$m | 0 - 5 % |
| $\geq 0,5$ und $\leq 1\ \mu$m | 15 - 35 % |
| $\geq 1$ und $\leq 3\ \mu$m | 30 - 55 % |

(fortgesetzt)

| Partikelgröße | Abscheidegrad (% bezogen auf die Gesamtpartikelanzahl der jeweiligen Partikelgröße) |
|---|---|
| $\geq 3$ und $\leq 5 \mu m$ | 60 - 90 % |
| $\geq 5$ und $\leq 10 \; \mu m$ | 85 - 98 % |
| $\geq 10 \; \mu m$ | 98 -100 % |

Alle vorgenannte Eigenschaften erfüllt beispielsweise das Filtervlies der Marke FIBROBAND®, der Filterklasse G 3 (DIN EN 779) der Fa. GEA Delbag Luftfilter GmbH.

Eine mögliche Ausführungsform von Gewebe- bzw. Vliesfiltern für das erfindungsgemäße Verfahren sind Schlauchfilter, z.B. in Reihen- oder Rundbauweise. Das angesaugte oder eingeblasene Rohgas (erfindungsgemäß normalerweise die Luft) tritt in der Regel von unten in die Schläuche ein, geht durch das Filtergewebe(-tuch), lagert dabei die enthaltenen Feinstpartikel dem Gewebe auf und verlässt das Filter oben als Reingas.

Eine raumsparendere Bauweise stellt das sogenannte Flächenfilter dar. Der Staub wird in Filtertaschen, welche aus mit Filtertuch bespannten Rahmen bestehen, zurückgehalten.

Die Abreinigung von verbrauchtem Filtergewebe kann z.B. durch in entgegengesetzter Weise strömende Pressluft und/oder durch Vibration vorgenommen werden. An die Stelle der Reinigung kann auch ein Ersatz mit Frischgewebe treten.

In anwendungstechnisch besonders vorteilhafter Weise wird man für das erfindungsgemäße Verfahren eine Ausführung als vollautomatisches Rollband-Filter wählen (z.B. gemäß Abb. 4 in Ullmanns Encyclopädie der technischen Chemie, 4. Auflage, Verlag Chemie (Weinheim), Bd. 2 (Verfahrenstechnik I), 1972, S. 238. Bei Erreichen eines einstellbaren Druckverlustanstiegs (in der Regel 0,05 bis 10 mbar bzw. 0,1 bis 5 mbar) wird über eine Druckdifferenzsteuerung der Motorantrieb in Tätigkeit gesetzt und soviel Filterband nachgeführt, bis der Sollwert des Druckverlustes (z.B. der zu frischem Filterband gehörige) wieder erreicht ist.

In einer möglichen Ausführungsform kann das Rollband-Filter auch stationär betrieben werden, d.h., es wird mit geringer Geschwindigkeit kontinuierlich frisches Filterband nachgeführt. Anstelle frisches Filterband druckverlustgesteuert nachzuführen, kann die Nachführung beim Rollbandfilter auch zeitgesteuert (z.B. alle 1-3 Tage) erfolgen.

Alternativ können für das erfindungsgemäße Verfahren auch Filterkerzen verwendet werden. Dies sind meist Filterelemente aus porösen, keramischen, metallischen oder Kunststoff-Formkörpern. Diese Filterelemente (Kerzen) sind einseitig geschlossene Zylinder, die am offenen Ende im Boden des sie aufnehmenden Apparates abgedichtet sind. Die Luft strömt durch diese Hohlzylinder von außen nach innen und wird dabei gereinigt.

In zweckmäßiger Weise wird die Luftfiltration beim erfindungsgemäßen Verfahren mehrstufig (z.B. zweistufig) durchgeführt. Dabei kommen für die erste Filtrierstufe insbesondere die folgenden Filtertypen in Betracht: Flächenfilter (z.B. Rollbandfilter), Kassettenfilter (Filterelement rechteckig), Patronenfilter (zylindrisches Filterelement) und Taschenfilter (Filterelement taschenförmig in rechteckigem Rahmen).

Für die zweite Filtrierstufe kommen insbesondere Taschenfilter und Kassettenfilter in Betracht.

In den höheren Filtrierstufen werden in zweckmäßiger Weise höhere Filterklassen ("feinere Filter") eingesetzt. Eine günstige Kombination wäre z.B. ein Taschenfilter für die erste Stufe und ein Kassettenfilter für die zweite Stufe. Eine weitere mögliche Kombination ist ein Rollbandfilter in der ersten Filtrierstufe und ein Taschenfilter oder Kassettenfilter für die zweite Filtrierstufe.

Eine geeignete Kombination für eine zweistufige Luftfiltration bildet auch die Paarung eines Filters vom Typ GAE YTS Roll Type (Hersteller: Airguard Industries) für die erste Filtrierstufe, sowie eines Filters vom Typ Koch Multicell 95 - K9242412 (Hersteller: Koch Filter Company) für die zweite Filtrierstufe.

Erfindungsgemäß bevorzugt sind generell die Filterklassen F6 (niederere Filterklasse) bis F9 (höhere Filterklasse) nach DIN EN 779 bzw. ASHRAE 52.1.

Auch ölbenetzte Metallfilter sind erfindungsgemäß geeignet. Insbesondere kommt für das erfindungsgemäße Verfahren z.B. ein Drehfilter mit ölbenetzten Metallfilterzellen in Betracht. Die Filterzellen werden dabei durch einen Motorantrieb kontinuierlich paternosterartig umgeführt. Beim Umlauf der Zellen werden sie durch einen Reinigungsbehälter geführt und hier ständig abgereinigt und neu benetzt.

Erfindungsgemäß wird normalerweise die Luft für sich filtriert. Selbstverständlich kann die Luft aber auch gemeinsam mit Kreisgas filtriert werden. Letzteres ist vor allem dann zweckmäßig, wenn Luft und Kreisgas in ein- und demselben Verdichter verdichtet werden.

Die Praxis hat jedoch gezeigt, dass eine Filtrierung des Kreisgases nicht unabdingbar, d.h. in der Regel nicht erforderlich ist.

Bei Bedarf können für das erfindungsgemäße Verfahren auch Filter verwendet werden, die sich gleichzeitig oder nachgeschaltet zur Abscheidung von Nebeln, d.h. Stoffen, die in Form von Tröpfchen, also flüssig, im Gas (z.B.

in der Luft) enthalten sind, eignen. Nach dem Aufprall auf das Filtermaterial agglomerieren die Nebel normalerweise, um anschließend aus den Filtern wegzufließen. Es können auch entsprechend konstruierte Schlauchfilter oder Kerzenfilter Verwendung finden.

Normalerweise führt ein Gehalt an Flüssigkeitströpfchen im zu filtrierenden Gas (z.B. der Luft) jedoch zum Verschmieren des z.B. Filtriergewebes.

Es ist daher erfindungsgemäß zweckmäßig, wenn die Temperatur des zu filtrierenden Gases (z.B. der Luft) nicht am Taupunkt liegt.

Vorzugsweise liegt sie wenigstens 30°C oberhalb derselben.

Um letzteres sicherzustellen, wird die zu filtrierende, dem Verdichter zuzuführende bzw. von diesem angesaugte Luft vorab ihrer Filtrierung in zweckmäßiger Weise über ein Heizregister geführt und entsprechend aufgewärmt.

In einfachster Weise besteht ein solches Heizregister aus einem Netzwerk aus heißen Wasserdampf führenden Rohren. Es könnte jedoch alternativ auch aus einem elektrisch beheizten Drahtgeflecht bestehen. Auch können zu diesem Zweck prinzipiell indirekte Wärmetauscher jedweder Art eingesetzt werden (z.B. Rohrbündelwärmetauscher).

Um zu verhindern, dass die Filtrier- und/oder Heizvorrichtung durch grobes mitangesaugtes Gut beschädigt wird, ist es zweckmäßig, vor diese ein grobmaschiges Raster zu schalten, das in entsprechender Weise abweisend wirkt.

Handelt es sich bei der Gasphasen-Partialoxidation um eine mehrstufig ausgeführte Partialoxidation, bei der dem Reaktionsgasgemisch zwischen den einzelnen Stufen verdichtete Sekundärluft zuzuführen ist, so ist mit dieser in vorteilhafter Weise ebenfalls erfindungsgemäß zu verfahren.

Erfindungsgemäß vorteilhaft wird man dem Reaktionsgasausgangsgemisch zuzusetzende Primärluft und dem Reaktionsgasgemisch zwischen den Stufen zuzusetzende Sekundärluft gemeinsam verdichten und gemeinsam der erfindungsgemäßen mechanischen Trennoperation unterwerfen.

Es überrascht, dass die vorteilhaften Auswirkungen der erfindungsgemäßen Verfahrensweise selbst dann noch zu spüren sind, wenn der Gehalt der im Reaktionsgasausgangsgemisch mitverwendeten Luft enthaltenen festen Feinstpartikel $\leq 150$ mg/m$^3$, oder $\leq 100$ mg/m$^3$, oder $\leq 50$ mg/m$^3$ beträgt. Die durchgeführten Untersuchungen haben gezeigt, dass der Gehalt der Luft an solchen festen Feinstpartikeln in der Regel $\geq 10$ mg/m$^3$ beträgt.

Das erfindungsgemäße Verfahren ist insbesondere für die sogenannten Hochlastverfahren von Bedeutung, bei denen die Belastung der Katalysatorbeschickung (insbesondere im Fall einer Festbettbeschickung) mit Reaktionsgasausgangsgemisch (Beschickungsgasgemisch) erhöht ist, wird bei ihnen pro Zeiteinheit doch eine erhöhte Menge an Reaktionsgasausgangsgemisch und damit eine erhöhte Menge an verdichteter Luft durch den Reaktor und die Katalysatorbeschickung geführt.

Das heißt, das erfindungsgemäße Verfahren ist besonders vorteilhaft für kontinuierliche heterogen katalysierte Gasphasen-Partialoxidationen wenigstens einer organischen Verbindung, bei denen die Belastung der Katalysatorbeschickung mit der wenigstens einen organischen Verbindung $\geq 120$ Nl/l Katalysatorbeschickung - h (Normallast erstreckt sich auf 60 bis < 120 Nl/l • h), bzw. $\geq 130$ Nl/l • h, oder $\geq 135$ Nl/l • h, oder $\geq 140$ Nl/l • h, oder $\geq 150$ Nl/l • h, oder $\geq 160$ Nl/l • h beträgt. Im Regelfall wir die vorgenannte Katalysatorbelastung $\leq 600$ Nl/l • h, häufig $\leq 400$ Nl/l • h bzw. $\leq 350$ Nl/l • h, oder $\leq 300$ Nl/l • h, bzw. $\leq 250$ Nl/l • h und manchmal $\leq 200$ Nl/l • h betragen.

Das vorgenannte gilt insbesondere dann, wenn die Gasphasen-Partialoxidation die Partialoxidation von Propen zu Acrolein oder die Gasphasen-Partialoxidation von Acrolein zu Acrylsäure ist (in entsprechender Weise sind dabei die vorgenannten Katalysatorbelastungen die Propen- bzw. Acroleinbelastung).

Die Durchführung dieser Hochlastpartialoxidationen kann im übrigen wie in den Schriften DE-A 4 431 957, DE-A 4 431 949, DE-A 19 948 241, DE-A 19 910 506, WO 00/53556, EP-A 1 106 598, DE-A 19 910 508, WO 00/53559, WO 00/53558, WO 00/53557 und DE-A 19 948 248 beschrieben erfolgen.

Als Reaktoren werden normalerweise Rohrbündelreaktoren eingesetzt.

Das Reaktionsgasausgangsgemisch der Propen-Gasphasen-Partialoxidation weist dabei üblicherweise folgende Zusammensetzung auf (Volumen (NI)-Verhältnis):

$$\text{Propen : Sauerstoff : inerte Gase (einschließlich Wasserdampf)}$$
$$= 1 : (1,0 \text{ bis } 3,0) : (5 \text{ bis } 25).$$

Das Reaktionsgasausgangsgemisch der Acrolein-Gasphasen-Partialoxidation weist dabei üblicherweise folgende Zusammensetzung auf (Volumen (NI)-Verhältnis):

Acrolein : Sauerstoff : Wasserdampf : inerte Gase
= 1 : (1 bis 3) : (0 bis 20) : (3 bis 30).

Beispiel und Vergleichsbeispiel

A) Allgemeine Beschreibung der Produktionsanlage

Das Beispiel und das Vergleichsbeispiel wurden in einer Produktionsanlage zur Herstellung von Acrylsäure durchgeführt, die das Verfahren gemäß der EP-A 784 046 abbildet. Sie besteht aus drei parallel betriebenen Produktionsstraßen, von denen jede zwei hintereinandergeschaltete Vielrohrreaktoren umfasst. Der erste Reaktor dient jeweils zur Partialoxidation von Propen zu Acrolein, der zweite zur Partialoxidation von Acrolein zu Acrylsäure.

Die Acrylsäure enthaltenden Reaktionsgase die aus den zweiten Reaktoren austraten wurden vereinigt und in einer Absorptionskolonne mit einem Gemisch aus Diphyl® und Dimethylphthalat gemäß der DE-A 19 606 877 im Gegenstrom absorbiert. Ein Teil des von Acrylsäure nahe zu frei gewaschenen Reaktionsgases (Kreisgas) welches hauptsächlich aus Stickstoff bestand, wurde als Bestandteil des Beschickungsgasgemisches für die ersten Reaktoren zurückgeführt, während der verbleibende Teil entsorgt wurde. Der aus der Absorptionskolonne entnommene flüssige Ablauf, der im wesentlichen aus dem Absorptionsmittel und Acrylsäure bestand, wurde seiner weiteren Aufarbeitung zugeleitet.

Das verwendete Propen war Propen der Qualität "chemical grade". Es enthielt $\geq$ 95,0 mol.% Propen und $\geq$ 4 mol-% Propan und wurde gasförmig einer Leitung entnommen. Das die ersten Reaktoren verlassende Reaktionsgasgemisch wurde abgekühlt und unter Zusatz von Sekundärluft in die zweiten Reaktoren geführt.

Die für das Reaktionsgasausgangsgemisch benötigte Primärluft und die benötigte Sekundärluft wurden gemeinsam von einem Turbo-Radialverdichter (Hersteller GHH, Typ GV 10/3) aus der Umgebung angesaugt und mit einem Druck von 2,5 bar dem Prozess zugeführt. Ein Heizregister (beheizt mit 4 bar Dampf) erwärmte die Luft vorab ihrer Verdichtung auf 35°C. Das Kreisgas wurde mit einem separaten Radialverdichter des Herstellers GHH, Typ GV 10/3 komprimiert und gefördert.

Die Reaktoren waren Rohrbündelreaktoren vom in den Schriften EP-A 700 893 und EP-A 700 714 beschriebenen Typ.

Die ersten Reaktoren umfassten jeweils einige tausend Rohre (gekühlt mit geschmolzenem Salz, Salzbadtemperatur ca. 290°C), ebenso wie die zweiten Reaktoren (gekühlt mit geschmolzenem Salz, Salzbadtemperatur ca. 260°C).

Die ersten Reaktoren waren mit einem Vollkatalysator gemäß Beispiel 1 der DE-A 10 046 957 und die zweiten Reaktoren waren mit einem Schalenkatalysator gemäß Herstellungsbeispiel 5 der DE-A 10 046 928 (Stöchiometrie: $Mo_{12}V_3W_{1,2}CU_{2,4}O_x$) beschickt.

Die Anlage wurde so betrieben, dass pro Stunde ca. 17 t Acrylsäure erzeugt wurden.

Die Zusammensetzung des Beschickungsgases für die ersten Reaktoren lautete:

6 bis 6,5 Vol.-% Propen chem. grade,
3 bis 3,5 Vol.-% $H_2O$,
0,3 bis 0,5 Vol.-% CO,
0,8 bis 1,2 Vol.-% $CO_2$
0,01 bis 0,04 Vol.-% Acrolein,
10,4 bis 10,7 Vol.-% $O_2$ und als
Restmenge ad 100 % molekularer Stickstoff.

Die für das den ersten Reaktoren zugeführte Reaktionsgasausgangsgemisch benötigte Luftmenge betrug 44000 $Nm^3$/h. Die für die zweiten Reaktoren benötigte Sekundärluftmenge betrug 12000 $Nm^3$/h. Die insgesamt zu verdichtende Luftmenge betrug somit 56000 $Nm^3$/h.

Unter diesen Bedingungen wurde die Produktionsanlage jeweils elf Monate mit folgenden Variationen betrieben:

B) Beispiel:

Hinter dem Heizregister befand sich noch vor dem Verdichter ein Rollbandfilter. Das (regenerierbare) Filtermaterial war eine Filtermatte der Fa. GEA Delbag Luftfilter GmbH (Berlin), Markenname FIBROBAND, Filterklasse G3 (DIN EN 779).

Das Filtermaterial bestand aus regellos gelagerten Polyesterfasern auf einer steifen, reißfesten Polyestergaze. Die Filterfläche betrug 10,8 m$^2$ (2,74 m Breite; 3,95 m Höhe).

Das Filtermaterial war so beschaffen, dass es in Abhängigkeit vom Volumenstrom V im Frischzustand die folgenden Druckverluste ΔP bedingte:

| V [Nm$^3$/hm$^2$] | ΔP [mbar] |
|---|---|
| 2000 | 0,1 |
| 3000 | 0,15 |
| 4000 | 0,22 |
| 5000 | 0,31 |
| 6000 | 0,42 |

Die Staubspeicherfähigkeit lag bei ca. 400 g/m$^2$.

Das Rollband wurde über eine Druckverlustmessung geregelt.

Sobald der Druckverlust einen Wert von 3 mbar erreichte, wurde das Filterband solange weitergerollt, bis der Druckverlust nur noch 2 mbar betrug (nur ein Teil der Filterfläche wurde so durch frische Fläche ersetzt, was den Gesamtverbrauch an Filtermaterial minderte).

Der mittlere Abscheidegrad betrug 88,1 %.

Laufradschwingungen waren beim Radialverdichter nicht beobachtbar.

C) Vergleichsbeispiel:

Es bestand freier Lufteinlass zum Radialverdichter, der nur aus Sicherheitsgründen mit einem grobmaschigen Sieb abgesichert war. Am Ende der Versuchsperiode waren beim Radialverdichter ein unrunder Lauf und Wellenschwingungen beobachtbar. Der Druckverlust in den Reaktoren stieg stärker als bei B). Die Selektivität der Acrylsäurebildung nahm leicht ab.

Die US-Provisional Patent Application No. 60/492726, eingereicht am 6. August 2003 und die US-Provisional Patent Application No. 60/530616, eingereicht am 19. Dezember 2003 sind eingefügt in die vorliegende Anmeldung durch Literaturhinweis.

**Patentansprüche**

1. Verfahren zum Betreiben einer kontinuierlichen heterogen katalysierten Gasphasen-Partialoxidation wenigstens einer organischen Verbindung in einem Oxidationsreaktor, dessen Beschickungsgasgemisch neben der wenigstens einen partiell zu oxidierenden Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfasst, bei dem sowohl als Sauerstoff - als auch als Inertgasquelle für das Beschickungsgasgemisch Luft mitverwendet wird, die zuvor in einem Verdichter von einem niederen Anfangsdruck auf einen höheren Enddruck verdichtet worden ist, **dadurch gekennzeichnet, dass** die Luft vorab ihres Eintritts in den Verdichter wenigstens einer mechanischen Trennoperation unterworfen wird, mit der in der Luft dispergierte Feststoffpartikel abgetrennt werden können.

2. Verfahren nach Anspruch 1, **dadurch gekennzeichnet, dass** es an einem Katalysatorfestbett durchgeführt wird.

3. Verfahren nach Anspruch 1 oder 2, **dadurch gekennzeichnet, dass** es bei einem atmosphärischen Überdruck von 0,2 bis 5 bar durchgeführt wird.

4. Verfahren nach einem der Ansprüche 1 bis 3, **dadurch gekennzeichnet, dass** die Luft mittels eines Radialverdichters verdichtet wird.

5. Verfahren nach einem der Ansprüche 1 bis 4, **dadurch gekennzeichnet, dass** an der heterogen katalysierten Gasphasen-Partialoxidation wenigstens eine eine ethylenisch ungesättigte Doppelbindung aufweisende chemische Verbindung beteiligt ist.

**6.** Verfahren nach einem der Ansprüche 1 bis 5, **dadurch gekennzeichnet, dass** die wenigstens eine organische Verbindung eine Verbindung aus der Gruppe umfassend Propen, Acrolein, iso-Buten, Methacrolein, iso-Butan und Propan ist.

**7.** Verfahren nach einem der Ansprüche 1 bis 6, **dadurch gekennzeichnet, dass** als mechanische Trennoperation eine Filtrierung angewendet wird.

**8.** Verfahren nach Anspruch 7, **dadurch gekennzeichnet, dass** als Filter ein Filtergewebe oder ein Faservlies verwendet wird.

**9.** Verfahren nach Anspruch 7 oder 8, **dadurch gekennzeichnet, dass** das Filter ein Faservlies auf Polyesterbasis ist.

**10.** Verfahren nach einem der Ansprüche 7 bis 9, **dadurch gekennzeichnet, dass** das Filter ein Rollbandfilter ist.

**11.** Verfahren nach einem der Ansprüche 7 bis 9, **dadurch gekennzeichnet, dass** das Filter ein Schlauchfilter ist.

**12.** Verfahren nach einem der Ansprüche 7 bis 11, **dadurch gekennzeichnet, dass** der Druckverlust des verwendeten frischen Filters bei einer Flächenbelastung von 5000 $Nm^3/m^2 \cdot h$ 0,01 bis 10 mbar beträgt.

**13.** Verfahren nach einem der Ansprüche 7 bis 12, **dadurch gekennzeichnet, dass** die Luft vorab der Filtrierung über eine Heizvorrichtung geführt wird.

**14.** Verfahren nach einem der Ansprüche 1 bis 13, **dadurch gekennzeichnet, dass** die kontinuierliche heterogen katalysierte Gasphasen-Partialoxidation wenigstens einer organischen Verbindung eine solche ist, bei der die Belastung der Katalysatorbeschickung mit der wenigstens einen organischen Verbindung ≥ 120 Nl/l•h beträgt.

**15.** Verfahren nach Anspruch 14, **dadurch gekennzeichnet, dass** die Belastung der Katalysatorbeschickung mit der wenigstens einen organischen Verbindung ≥ 130 Nl/l•h beträgt.

**16.** Verfahren nach Anspruch 14, **dadurch gekennzeichnet, dass** die Belastung der Katalysatorbeschickung mit der wenigstens einen organischen Verbindung ≥ 140 Nl/l•h beträgt.

**17.** Verfahren nach einem der Ansprüche 1 bis 16, **dadurch gekennzeichnet, dass** als mechanische Trennoperation eine Abtrennung in einem Zyklon angewendet wird.

**Claims**

**1.** A process for operating a continuous heterogeneously catalyzed gas phase partial oxidation of at least one organic compound in an oxidation reactor whose charging gas mixture, in addition to the at least one compound to be partially oxidized and molecular oxygen as the oxidant, comprises at least one diluent gas which is substantially inert under the conditions of the heterogeneously catalyzed gas phase partial oxidation, by using air, both as the oxygen source and the inert gas source for the charging gas composition, which has been compressed beforehand in a compressor from a low starting pressure to a higher final pressure, which comprises subjecting the air, before its entry into the compressor, to at least one mechanical separating operation by which solid particles dispersed in the air can be removed.

**2.** The process according to claim 1, which is carried out over a fixed catalyst bed.

**3.** The process according to claim 1 or 2, which is carried out at a superatmospheric pressure of from 0.2 to 5 bar.

**4.** The process according to any of claims 1 to 3, wherein the air is compressed by means of a radial compressor.

**5.** The process according to any of claims 1 to 4, wherein at least one chemical compound having an ethylenically unsaturated double bond is involved in the heterogeneously catalyzed gas phase partial oxidation.

**6.** The process according to any of claims 1 to 5, wherein the at least one organic compound is a compound from the group consisting of propene, acrolein, iso-butene, methacrolein, isobutane and propane.

**7.** The process according to any of claims 1 to 6, wherein the mechanical separating operation employed is a filtration.

**8.** The process according to claim 7, wherein the filter used is a filter fabric or a fiber web.

**9.** The process according to claim 7 or 8, wherein the filter is a fiber web based on polyester.

**10.** The process according to any of claims 7 to 9, wherein the filter is a roll belt filter.

**11.** The process according to any of claims 7 to 9, wherein the filter is a bag filter.

**12.** The process according to any of claims 7 to 11, wherein the pressure drop of the fresh filter used is from 0.01 to 10 mbar at a superficial velocity of 5000 $m^3$ (STP)/$m^2 \cdot$ h.

**13.** The process according to any of claims 7 to 12, wherein the air is conducted through a heating apparatus before the filtration.

**14.** The process according to any of claims 1 to 13, wherein the continuous heterogeneously catalyzed gas phase partial oxidation of at least one organic compound is one in which the hourly space velocity of the at least one organic compound on the catalyst charge is $\geq$ 120 1 (STP)/l•h.

**15.** The process according to claim 14, wherein the hourly space velocity of the at least one organic compound on the catalyst charge is $\geq$ 130 1 (STP)/l•h.

**16.** The process according to claim 14, wherein the hourly space velocity of the at least one organic compound on the catalyst charge is $\geq$ 140 1 (STP)/l•h.

**17.** The process according to any of claims 1 to 16, wherein the mechanical separating operation employed is a separation in a cyclone.

**Revendications**

**1.** Procédé d'exploitation d'une oxydation partielle continue en phase gazeuse sous catalyse hétérogène d'au moins un composé organique dans un réacteur d'oxydation, dont le mélange gazeux d'alimentation comprend, outre le ou les composés à oxyder partiellement et de l'oxygène moléculaire en tant qu'agent d'oxydation, au moins un gaz diluant essentiellement inerte dans les conditions de l'oxydation partielle en phase gazeuse sous catalyse hétérogène, selon lequel de l'air qui a auparavant été comprimé d'une pression initiale basse à une pression finale plus élevée dans un compresseur est utilisé aussi bien en tant qu'oxygène qu'en tant que source de gaz inerte pour le mélange gazeux d'alimentation, **caractérisé en ce que** l'air est soumis, avant son entrée dans le compresseur, à au moins une opération de séparation mécanique par le biais de laquelle les particules solides dispersées dans l'air peuvent être séparées.

**2.** Procédé selon la revendication 1, **caractérisé en ce qu'**il est réalisé sur un lit catalytique solide.

**3.** Procédé selon la revendication 1 ou 2, **caractérisé en ce qu'**il est réalisé à une surpression atmosphérique de 0,2 à 5 bars.

**4.** Procédé selon l'une quelconque des revendications 1 à 3, **caractérisé en ce que** l'air est comprimé à l'aide d'un compresseur radial.

**5.** Procédé selon l'une quelconque des revendications 1 à 4, **caractérisé en ce qu'**au moins un composé chimique comportant une double liaison éthyléniquement insaturée participe à l'oxydation partielle en phase gazeuse sous catalyse hétérogène.

**6.** Procédé selon l'une quelconque des revendications 1 à 5, **caractérisé en ce que** l'au moins un composé organique est un composé du groupe comprenant le propène, l'acroléine, l'iso-butène, la méthacroléine, l'iso-butane et le propane.

**7.** Procédé selon l'une quelconque des revendications 1 à 6, **caractérisé en ce qu'**une filtration est utilisée en tant qu'opération de séparation mécanique.

**8.** Procédé selon la revendication 7, **caractérisé en ce qu'**un tissu filtrant ou un non-tissé fibreux est utilisé en tant que filtre.

**9.** Procédé selon la revendication 7 ou 8, **caractérisé en ce que** le filtre est un non-tissé fibreux à base de polyester.

**10.** Procédé selon l'une quelconque des revendications 7 à 9, **caractérisé en ce que** le filtre est un filtre à rouleaux.

**11.** Procédé selon l'une quelconque des revendications 7 à 9, **caractérisé en ce que** le filtre est un filtre à manche.

**12.** Procédé selon l'une quelconque des revendications 7 à 11, **caractérisé en ce que** la perte de charge du filtre frais utilisé avec une charge de surface de 5 000 $Nm^3/m^2 \cdot h$ est de 0,01 à 10 mbars.

**13.** Procédé selon l'une quelconque des revendications 7 à 12, **caractérisé en ce que** l'air est passé sur un dispositif chauffant avant la filtration.

**14.** Procédé selon l'une quelconque des revendications 1 à 13, **caractérisé en ce que** l'oxydation partielle continue en phase gazeuse sous catalyse hétérogène d'au moins un composé organique est telle que la charge du garnissage catalytique avec le ou les composés organiques est $\geq$ 120 Nl/l·h.

**15.** Procédé selon la revendication 14, **caractérisé en ce que** la charge du garnissage catalytique avec le ou les composés organiques est $\geq$ 130 Nl/l·h.

**16.** Procédé selon la revendication 14, **caractérisé en ce que** la charge du garnissage catalytique avec le ou les composés organiques est $\geq$ 140 Nl/l·h.

**17.** Procédé selon l'une quelconque des revendications 1 à 16, **caractérisé en ce qu'**une séparation dans un cyclone est utilisée en tant qu'opération de séparation mécanique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 2351151 A
- DE 2526238 A
- EP 92097 A
- EP 58927 A
- DE 4132263 A
- DE 4132684 A
- DE 4022212 A
- DE 2106796 A
- DE 1624921 A
- GB 1464198 A
- GB 1291354 A
- DE S1254137 A
- DE 2159346 A
- EP 372972 A
- WO 890710 A
- DE 4311608 A
- DE 10131297 A
- EP 1090684 A
- EP 608838 A
- DE 10046672 A
- EP 529853 A
- WO 0196270 A
- DE 10028582 A
- EP 1180508 A
- DE 19902562 A
- EP 990636 A
- EP 1106598 A
- DE 19606877 A
- DE 19631645 A
- EP 982289 A

- DE 19740253 A
- EP 982287 A
- EP 1041062 A
- EP 778255 A
- EP 695736 A
- DE 19501325 A
- EP 925272 A
- DE 19927624 A
- DE 19948248 A
- DE 19948241 A
- DE 19910508 A
- DE 10313210 A
- DE 10313214 A
- DE 10313213 A
- DE 19910506 A
- DE 10259023 A
- DE 4431957 A
- DE 4431949 A
- WO 0053556 A
- WO 0053559 A
- WO 0053558 A
- WO 0053557 A
- EP 784046 A
- EP 700893 A
- EP 700714 A
- DE 10046957 A
- DE 10046928 A
- US 60492726 B
- US 60530616 B

### In der Beschreibung aufgeführte Nicht-Patentliteratur

- **Beyer.** Lehrbuch der organischen Chemie. Hirzel Verlag, 1973, vol. 17, 261